(19)

**Europäisches Patentamt**
**European Patent Office**
**Office européen des brevets**

(11) **EP 3 827 672 A1**

(12) **EUROPEAN PATENT APPLICATION**

(43) Date of publication:
**02.06.2021 Bulletin 2021/22**

(21) Application number: **19212747.0**

(22) Date of filing: **30.11.2019**

(51) Int Cl.:
*A23L 3/3472* (2006.01)  *A61K 8/9794* (2017.01)
*A61K 36/899* (2006.01)  *A61P 17/10* (2006.01)
*A61P 17/18* (2006.01)  *A61Q 17/04* (2006.01)
*A61Q 19/08* (2006.01)  *A23L 29/206* (2016.01)
*A23L 33/105* (2016.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **29.11.2019   PT 2019616087**

(71) Applicants:
• **Universidade Católica Portuguesa - UCP**
  **4169-005 Porto (PT)**

• **Amyris Bio Products Portugal, Unipessoal, LDA**
  **4169-005 Porto (PT)**

(72) Inventors:
• **Estevez Pintado, Maria Manuela**
  **4169-005 Porto (PT)**
• **Mendes Ferreira Monteiro Madureira, Ana Raquel**
  **4169-005 Porto (PT)**
• **da Silva Oliveira, Ana Lúcia**
  **4169-005 Porto (PT)**

(74) Representative: **Patentree**
**Edificio Net**
**Rua de Salazares, 842**
**4149-002 Porto (PT)**

(54) **MULTIFUNCTIONAL EXTRACTS OF SUGARCANE STRAW OR BAGASSE AND USES THEREOF**

(57) Provided herein are extracts of sugarcane straw or bagasse, methods of preparing the extracts, and the use of the extracts in compositions that have antioxidant activity, anti-inflammation activity, and antimicrobial activity that can be used as multifunctional ingredients. Further provided are methods of using the extracts to treat or ameliorate conditions involving oxidation, inflammation, skin and food enzymes inhibition activity capacity and microbial growth.

EP 3 827 672 A1

**Description**

**TECHNICAL FIELD**

The present disclosure relates to multifunctional extracts of sugarcane straw or bagasse, methods of preparing the extracts, and their use as antioxidants, anti-inflammation agents, skin and food enzymes inhibition activity capacity and anti-microbial agents.

**BACKGROUND**

Sugarcane straw and bagasse are by-products of sugar production and industrial fermentation. In Brazil and India alone, the sugarcane industry generates 800 million metric tons of these sugarcane by-products each year. Bagasse and straw are currently considered waste and sugarcane producing companies incur significant costs in their disposal. Novel high value uses of these by-products would yield environmental benefits as well as financial gain for the companies that produce the by-products (from additional revenue from the new uses as well from the reduction or elimination of the costs associated with the disposal of the by-products).

Sugarcane straw and bagasse are known to contain a variety of commercially interesting compounds including cellulose, hemicellulose, lignin, sugars, starch, wax, amino acids, organic acids, minerals, and phenolic compounds. These types of extracts are generally referred to as antioxidant, but they possess other properties and can be used to develop multifunctional ingredients for cosmetic and food applications.

These facts are disclosed in order to illustrate the technical problem addressed by the present disclosure.

**GENERAL DESCRIPTION**

The present disclosure describes multifunctional extracts of sugar cane straw or bagasse, methods of preparing the extracts, and methods of using the extracts. In particular, the extracts have multiple biological activities including antioxidant activity, anti-inflammation activity, skin and food enzymes inhibition activity capacity and antimicrobial activity.

In an aspect, the present disclosure describes a method of preparing an extract from sugarcane bagasse involving the steps of drying the bagasse; milling the dry bagasse; mixing the milled bagasse with a solution comprising a solvent; stirring the bagasse solvent mixture; separating a liquid fraction and a solid fraction of the mixture; and concentrating the liquid fraction and drying the liquid to obtain the extract.

In an embodiment the bagasse is dried at a temperature greater than 30°C. In another embodiment the bagasse is dried at a temperature of about 40°C. In yet another embodiment the dried bagasse is milled to an average size of ranging from about 2 mm to 4 mm. In additional embodiments the solvent is selected from acetone, dichloromethane, ethanol, and methanol or a combination thereof. In a preferred embodiment the solution comprising solvent comprises ethanol. In an embodiment of the invention the bagasse ethanol mixture is stirred for at least 24 hours. In another embodiment the bagasse ethanol mixture is stirred at room temperature, preferably a temperature ranging from 20 °C to 22 °C. In further embodiments the liquid and solid fractions are separated by filtration through gauze. In another embodiment the liquid fraction is concentrated and not lyophilized.

In yet another aspect of the present disclosure describes a method of preparing an extract from sugarcane straw involving the steps of drying the straw; milling the dry straw; mixing the milled straw with a solution comprising a solvent; stirring the straw solvent mixture; separating a liquid fraction and a solid fraction of the mixture; and lyophilizing the liquid fraction to obtain the extract. In another embodiment the liquid fraction is concentrated and not lyophilized.

In an embodiment the straw is dried at a temperature greater than 30°C. In another embodiment the straw is dried at a temperature of about 40°C. In another embodiment the dried straw is milled to an average size ranging from of about 2 mm to 4 mm. In yet another embodiment the solvent is selected from acetone, dichloromethane, ethanol, and methanol or a combination thereof. In a preferred embodiment the solvent comprises ethanol. In further embodiments the straw solvent mixture is stirred for at least 24 hours. In another embodiment the straw ethanol mixture is stirred at room temperature, preferably at a temperature ranging from 20 °C to 22 °C. In other embodiments the liquid and solid fractions are separated by filtration through gauze. In another embodiment the liquid fraction is concentrated and not lyophilized.

In another aspect the present disclosure describes a multifunctional extract made by any of the methods disclosed herein. In another embodiment, the invention is a cosmetic ingredient containing the extract. In a further embodiment the cosmetic comprises at least 1.25 mg/mL of the extract.

In another aspect the present disclosure describes a method of reducing bacteria on skin of a subject involving the step of applying the cosmetic containing the extract to the skin of the subject. In another embodiment the present disclosure describes an anti-inflammatory composition comprising the extract. In yet another embodiment the anti-inflammatory composition contains at least 1.25 mg/mL of the extract. In further embodiments the invention provides a method of reducing inflammation in a subject involving the step of administering the anti-inflammatory composition to the subject. In another embodiment the administering comprises topically applying the anti-inflammatory composition.

In another aspect the present disclosure describes a sunscreen containing the extract. In an embodiment the anti-ageing ingredient contains at least 1.25 mg/mL of the extract. In another embodiment the present disclosure describes a method of protecting skin of a subject from UV light damage involving the step of applying the ingredient to the skin of the subject prior to exposure to UV light. In another embodiment the present disclosure describes a method of protection the skin from signs of ageing.

In yet another aspect the present disclosure describes a multifunctional sugar cane bagasse extract containing from about 2% to 8% cellobiose, from about 2% to 10% glucose, not more than about 2% xylose, not more than 1% galactose, not more than 0.1% arabinose, not more than 15% fructose, and not more than 6% mannitol. In an embodiment the sugar cane bagasse extract also contains not more than 4% malic acid, not more than 5% succinic acid, and not more than 3% formic acid. In yet another embodiment the sugar cane bagasse extract contains not more than 1% chlorogenic acid, not more than 1% trans-5-caffeoylquinic acid, not more than 10% trans-3-feruloylquinic acid, not more than 1% feruylquinic acid, not more than 1% feruylglucaric acid, not more than 1% p-coumaric acid, not more than 1% trans-ferulic acid, not more than 1% diosmetin-6-C-glucoside, not more than 1% tricin-7-O-glucuronidesulfate, and not more than 1% pinellic acid.

In a further aspect the present disclosure describes a multifunctional sugar cane straw extract containing not more than 2% cellobiose, from about 1% to 8% glucose, not more than about 1% xylose, not more than 2% arabinose, not more than 12% fructose, and not more than 6% mannitol. In an embodiment the sugar cane straw extract also contains from about 5 % to about 12% malic acid, from about 3% and 20% succinic acid, and not more than 10% formic acid. In another embodiment the sugar cane straw extract contains not more than 1% chlorogenic acid, not more than 1% trans-5-caffeoylquinic acid, not more than 1% trans-3-feruloylquinic acid, not more than 1% feruylquinic acid, not more than 1% feruylglucaric acid, not more than 1% p-coumaric acid, not more than 1% trans-ferulic acid, not more than 1% diosmetin-6-C-glucoside, not more than 1% tricin-7-O-glucuronidesulfate, and not more than 1% pinellic acid.

In an embodiment, the present disclosure describes a food product containing a multifunctional sugar cane bagasse or multifunctional sugar cane straw extract. In a further embodiment, the food product containing the sugar cane bagasse extract or sugar cane straw extract is an animal feed.

In an embodiment, the method of preparing an extract from sugarcane bagasse or sugarcane straw comprises:

obtaining a dried sugarcane bagasse or a dried sugarcane straw;
milling the dried bagasse or the dried straw;
mixing the milled bagasse or straw with a solution comprising a solvent to form a mixture;
stirring the mixture;
separating liquid fraction and solid fraction of the mixture;
concentrating the liquid fraction to obtain an extract;
optionally drying the liquid fraction to obtain a dry extract.

In an embodiment, the bagasse or the straw are dried at a temperature not less than 30 °C, preferably at a temperature of about 40 °C.

In an embodiment, the dried bagasse or the dried straw is milled to a size ranging from 2 mm to 4 mm.

In an embodiment, the solvent is selected from acetone, dichloromethane, ethanol, methanol, or combinations thereof.

In an embodiment, the solution comprising solvent comprises ethanol.

In an embodiment, the mixture is stirred for at least 24 hours.

In an embodiment, the mixture is stirred at a temperature ranging from 20 °C to 22 °C.

In an embodiment, the liquid fraction and the solid fractions are separated by filtration, preferably filtration through gauze.

In an embodiment, an extract is obtained by the method of preparing an extract from sugarcane bagasse or sugarcane straw as described above.

In an embodiment, the extract is for use in medicine or veterinary.

In an embodiment, the extract is for use in the treatment or prevention of inflammatory diseases or inflammatory symptoms, in particular in skin inflammatory diseases such as acne.

In an embodiment, the extract is for use in the inhibition or reduction of skin enzymes activity, as anti-ageing agent, the skin protection against UV light damage, as antioxidant, as an anti-microbial agent and/or as an anti-acne agent.

In an embodiment, the extract comprises from about 2% (wt/wt) to 8% (wt/wt) cellobiose, from about 2% (wt/wt) to 10% glucose(wt/wt), not more than about 2%(wt/wt) xylose, not more than 1% (wt/wt) galactose, not more than 0.1%(wt/wt) arabinose, not more than 15% (wt/wt) fructose, and not more than 6%(wt/wt) mannitol.

In an embodiment, the extract further comprises not more than 4% (wt/wt) malic acid, not more than 5% (wt/wt) succinic acid, and not more than 3% (wt/wt) formic acid.

In an embodiment, the extract further comprises not more than 1% (wt/wt) chlorogenic acid, not more than 1% (wt/wt) trans-5-caffeoylquinic acid, not more than 10% (wt/wt) trans-3-feruloylquinic acid, not more than 1% (wt/wt) feruylquinic acid, not more than 1% (wt/wt) feruylglucaric acid, not more than 1% (wt/wt) p-coumaric acid, not more than 1% (wt/wt) rans-ferulic acid, not more than 1% (wt/wt) diosmetin-6-C-glucoside, not more than 1% (wt/wt) tricin-7-O-glucuronidesulfate, and not more than 1% (wt/wt) pinellic acid.

In an embodiment, the extract further comprises not more than 2% (wt/wt) cellobiose, from about 1%(wt/wt) to 8% (wt/wt) glucose, not more than about 1% (wt/wt) xylose, not more than 2% (wt/wt) arabinose, not more than 12% (wt/wt) fructose, and not more than 6% (wt/wt) mannitol.

In an embodiment, the extract further comprises about 5 % (wt/wt) to 12% (wt/wt) malic acid, from about 3% (wt/wt) to about 20% (wt/wt) succinic acid, and not more than 10% (wt/wt) formic acid.

In an embodiment, the extract further comprises not more than 1% (wt/wt) chlorogenic acid, not more than 1% (wt/wt) trans-5-caffeoylquinic acid, not more than 1% (wt/wt) trans-3-feruloylquinic acid, not more than 1% (wt/wt) feruylquinic acid, not more than 1% (wt/wt) feruylglucaric acid, not more than 1% (wt/wt) p-coumaric acid, not more than 1% (wt/wt) trans-ferulic acid, not more than 1% (wt/wt) diosmetin-6-C-glucoside, not more than 1% (wt/wt) tricin-7-O-glucuronidesulfate, and not more than 1% (wt/wt) pinellic acid.

In an embodiment, a composition comprises an active amount of the extract as described above and suitable excipients is obtained, in particular a pharmaceutical composition or a cosmetic composition.

In an embodiment, the concentration of the extract in the composition is at least 1.25 mg/mL.

In an embodiment, the composition is for topical administration, preferably the topical composition comprises a penetration enhancer.

In an embodiment, the composition further comprises an active agent.

In an embodiment, the extract is used as a preservative, in particular as a cosmetic preservative or food preservative.

In an embodiment, a foodstuff or a food composition comprising the extract as described above is obtained.

In an embodiment, a method of reducing inflammation in a subject comprises administering the anti-inflammatory composition comprising the extract to the subject.

In an embodiment, a method of reducing inflammation in a subject comprises topically applying the anti-inflammatory composition comprising the extract to the subject.

In an embodiment, a method of reducing the activity of skin enzymes in a subject comprises administering a composition comprising the extract to the subject.

In an embodiment, a method of protecting skin of a subject from UV light damage comprises applying a composition comprising the extract to the skin of the subject prior to exposure to UV light.

In an embodiment, a method of treating acne in a subject comprises administering a composition comprising an effective amount the extract or a mixture thereof to the subject.

In an embodiment, the administering step comprises topically applying a composition comprising an effective amount the extract to the subject.

## BRIEF DESCRIPTION OF THE DRAWINGS

The following figures provide preferred embodiments for illustrating the disclosure and should not be seen as limiting the scope of invention.

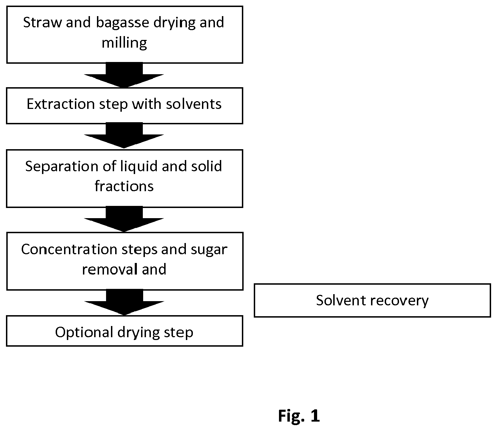

Figure 1 is a flow diagram of the process used to prepare the extract from sugarcane straw and sugarcane bagasse.

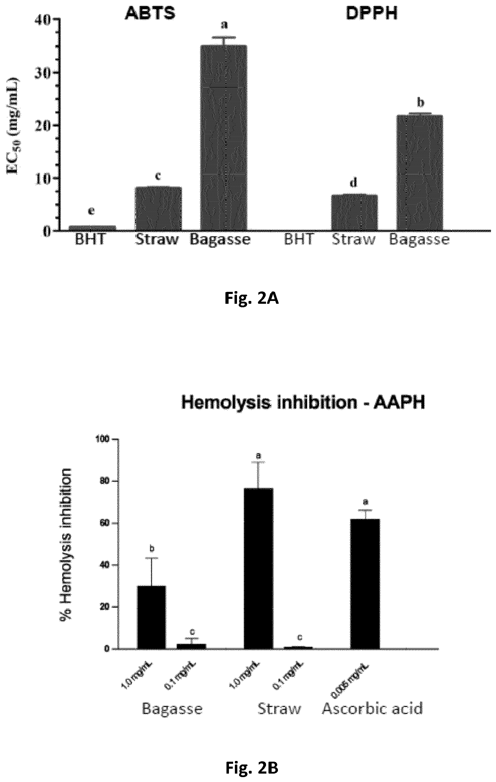

Figures 2A and 2B are a set of graphs showing that straw and bagasse extracts have antioxidant activity. Figure 2A shows the antioxidant activity of straw and bagasse extracts compared to a BHT control in the 2,2'-Azino-bis (3-ethylbenzothiazoline-6-sulfonic acid) radical cation (ABTS) assay and 1,1-Diphenyl-2-picrylhydrazyl radical; 2,2-Diphenyl-1-picrylhydrazyl free radical (DPPH) assay. Figure 2B shows that straw and bagasse extracts inhibit hemolysis in an AAPH assay compared to an ascorbic acid control.

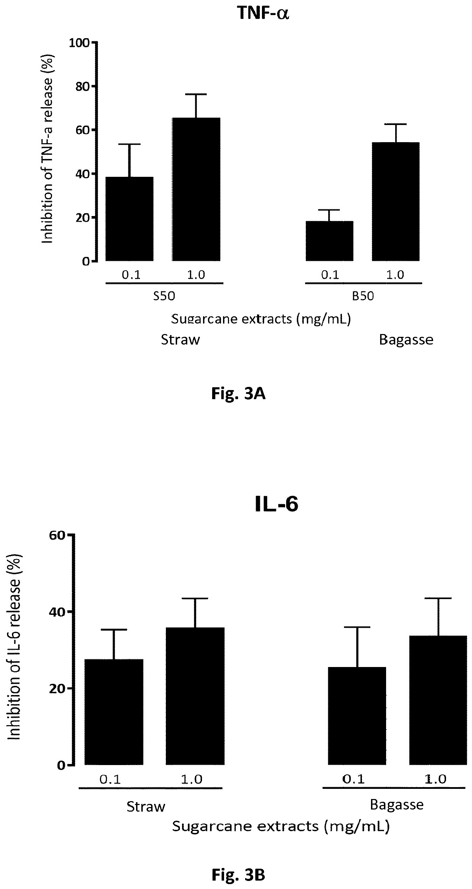

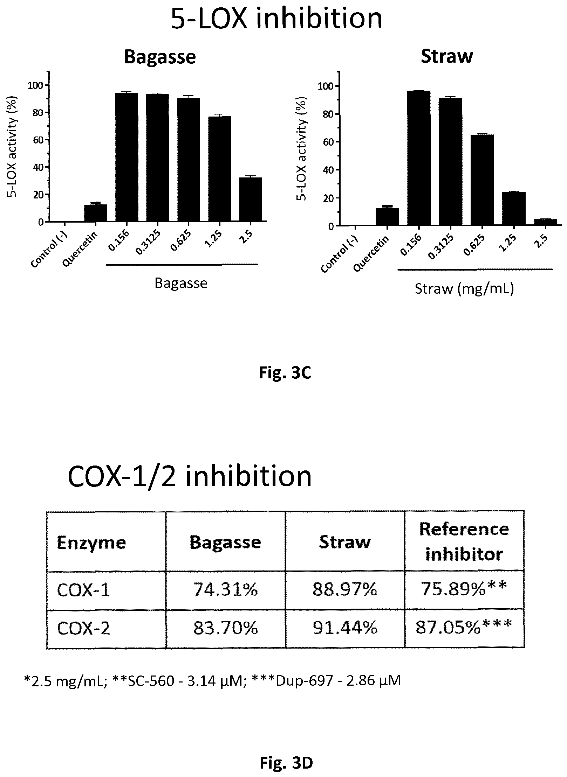

Figures 3A-3D are graphs showing the anti-inflammatory activity of sugarcane extracts. Figure 3A shows extract ability to inhibit TNF-$\alpha$ release, Figure 3B shows extract ability to inhibit IL-6 release, Figure 3C shows extract ability to inhibit enzyme 5-lipoxygenase (5-LOX), and Figure 3D shows extract ability to inhibit cyclooxygenase isoenzymes, COX-1 and COX-2.

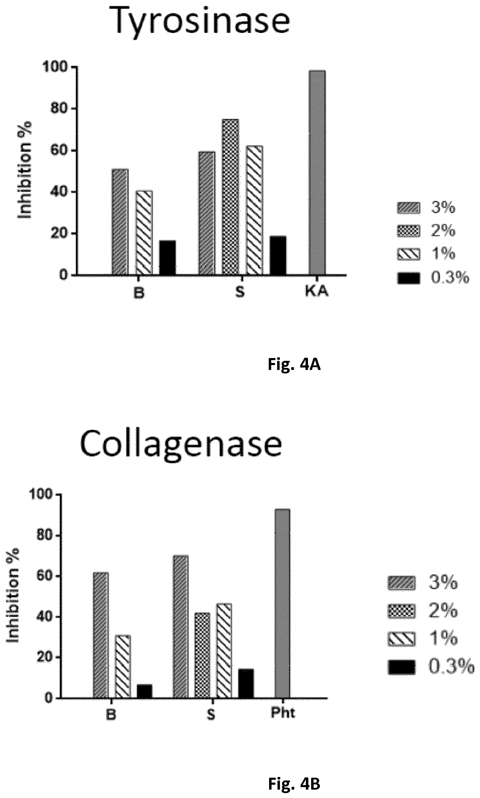

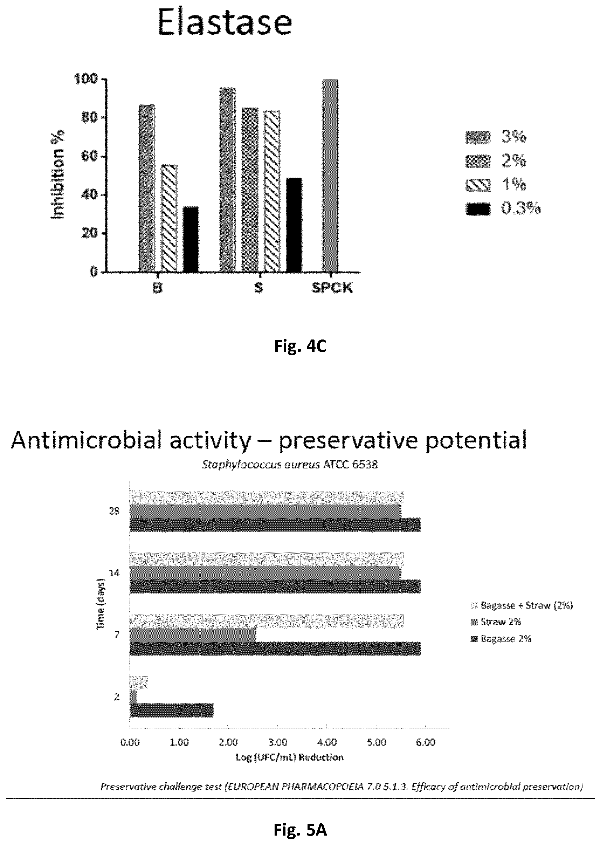

Figures 4A-4C are graphs showing the ability of straw and bagasse extracts ability to inhibit the enzymes tyrosinase, collagenase, and elastase respectively. Figure 4A shows extract inhibition of tyrosinase with kojic acid (KA) as a control, Figure 4B shows extract inhibition of collagenase with Phenanthroline (Pht) as a control, and Figure 4C shows extract inhibition of elastase with SPCK as a control.

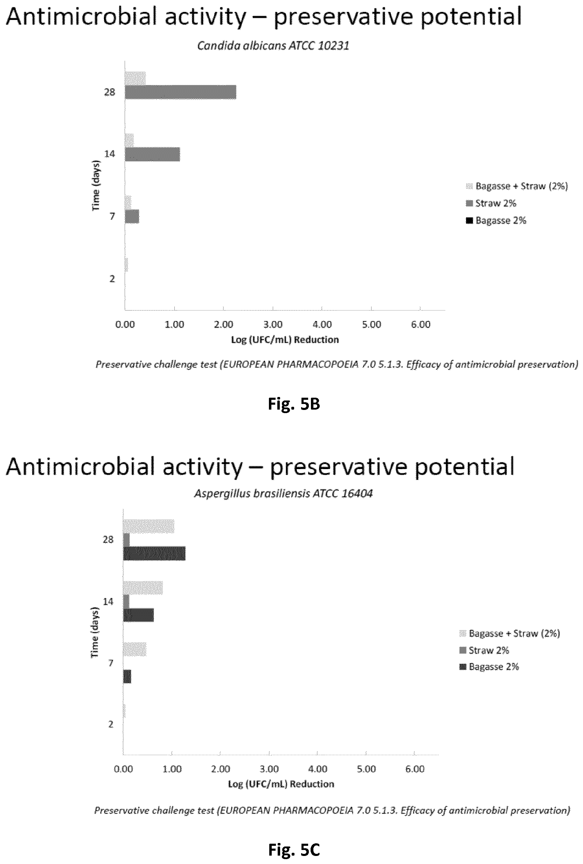

Figures 5A, 5B, 5C, and 5D are graphs showing the antimicrobial activity of straw and bagasse extracts. The graphs show the reduction of cell growth caused by the extracts on *S. aureus* (Fig. 5A), *C. albicans* (Fig. 5C), and *A. brasiliensis* (Fig. 5D).

## DETAILED DESCRIPTION

As used herein "bagasse" refers to the residue left after the extraction of juice from sugarcane or sorghum stalks.

As used herein "sugarcane straw" refers to the portion of the sugarcane plant that remains after the sugarcane stalks are harvested.

As used herein "multifunctional" and "multifunctional extract" refers to the property of an extract having multiple biological activities. A non-limiting set of biological activities includes antioxidant activity, anti-inflammation activity, skin and food enzymes inhibition activity capacity and antimicrobial activity. Such a multifunctional extract has the benefit of conferring multiple biological properties to any formulation of which it is an ingredient.

As used herein an "effective amount" means an amount necessary to at least partly attain the desired response, or to delay the onset or inhibit progression or halt altogether, the onset or progression of a condition being treated. The amount varies depending upon the health and physical condition of the individual to be treated, the taxonomic group of individual to be treated, the degree of protection desired, the formulation of the composition, the assessment of the medical situation, and other relevant factors. It is expected that the amount will fall in a relatively broad range that can be determined through routine trials.

As used herein, "subject" or "patient" is an organism that is treated using one of the methods of the present disclosure. In some embodiment, the subject is a mammalian subject, such as a human or a domestic animal.

A "water-solubilized" formulation, as used herein, includes the natural hydrophilic and lipophilic compounds, phenolic compounds, organic acids, oligo and polysaccharides, minerals, as other metabolites and compositional components, and water (e.g. a water containing liquid) or not, but often does not include organic solvents (e.g. ethanol). In some embodiments, the water solubilized formulation is a water-soluble formulation or a powder.

As used herein, the term "about" will be understood by persons of ordinary skill in the art and will vary to some extent on the context in which is used. If there are uses of the term which are not clear to persons of ordinary skill in the art given the context in which is used, 'about' may mean up to plus or minus 20% of the term.

As used herein, the term "ointment" may be any commonly known and commercially available ointments.

As used herein, the term "cosmetic" or "cosmetic composition" means any substance or mixture intended to be placed in contact with various external parts of a subject's body (epidermis, hair systems, nails, lips, and external genital organs) or with the teeth and the mucous membranes of the oral cavity with the intent primarily to clean, perfume, or changing the appearance of the subject's body, teeth, or mucous membranes of the oral cavity.

As used herein, the term "food" or "food product" means any substance or composition that can be eaten or drunk by animals including humans for nutrition or pleasure. Where the food or food product is used by non-human animals the food or food product may also be referred to as feed.

Bagasse and sugarcane straw can be dried using any suitable method to reduce the moisture content. In an embodiment, bagasse or straw are dried in the open air, also referred to a solar or passive drying. The bagasse or straw can be set on screens that allow airflow from both sides of the material. In other embodiments, bagasse and straw can be dried in a dryer or kiln. Illustrative examples of dryers include counter current flow dryers, rotary drum dryers, and pneumatic dryers.

Rotary dryers typically comprise a large rotating cylindrical tube. Bagasse or straw enters the dryer tube, and as the dryer tube rotates, the bagasse or straw is lifted by a series of internal fins lining the inner wall of the tube. As the bagasse or straw falls back to the floor of the dryer tube it passes through a hot gas stream. In some embodiments the gas stream is a mixture of air and combustion gases from a burner. In other embodiments, the gas stream comprises gas that was preheated. In an embodiment, the rotary dryer tube is situated at an angle that allows the dried material to pass through the tube by gravity.

In an embodiment the dryer is a counter current dryer. A counter current dryer uses drying gases that flows in the opposite direction of the bagasse or straw material being dried. In counter current dryers the wettest bagasse or straw contacts the coolest drying gas and will contact the hottest gases at the discharge end of the dryer. This process increases the thermal efficiency of the bagasse or straw drying process.

In an embodiment the dryer is a pneumatic dryer, also referred to as a flash dryer. In a typical pneumatic dryer, the bagasse or straw is introduced by the wet material feeder. A burner or heating unit introduces heated gases into the dryer, and the heated gases combine with the bagasse or straw in the cyclone which is a large tank that allows the heated gases to remove the moisture from the bagasse or straw. The dried material is then expelled from the cyclone and captured by a filter bag or capture chamber.

The bagasse or straw can be milled by any of the methods that are known in the art. Milling is the mechanical pre-treatment that breaks down the structure of the bagasse and straw materials. Ball milling is a method where the bagasse or straw is introduced into a hollow vessel that contains several balls. Rotation of the hollow vessel causes the balls to crush and grind the bagasse or straw and reduces the particle size. Illustrative alternative milling processes include the use of cutter mills and wet disk milling.

A variety of solvents can be used to prepare the extracts from bagasse or straw. Illustrative examples of appropriate solvents include acetone, dichloromethane, ethanol, or methanol. In embodiments with ethanol or methanol, the solvents can be diluted with water. In an embodiment the solvent is ethanol and water comprising at least 20%, 30%, 40%, 50%, 60%, 70%, 75%, 80%, 85%, 90%, 95%, 96%, 97%, 98%, or 99% ethanol. In preferred embodiments the solvent comprises ethanol and water comprising at least 80% ethanol. In other embodiments the solvent is methanol and water comprising at least about 20%, 30%, 40%, 50%, 60%, 70%, 75%, 80%, 85%, 90%, 95%, 96%, 97%, 98%, or 99% methanol.

During the extraction process a variety of ratios of bagasse or straw biomass to solvent can be used. In an embodiment, the ratio of bagasse or straw biomass to solvent in the extraction step is at or about 1:1 (w/v), 1:2 (w/v), 1:4 (w/v), 1:8 (w/v), 1:10 (w/v), 1:15 (w/v), 1:16 (w/v), 1:17 (w/v), 1:18 (w/v), 1:19 (w/v), 1:20 (w/v), 1:21 (w/v). 1:22 (w/v), 1:23 (w/v), 1:24 (w/v), 1:25 (w/v), 1:30 (w/v), 1:40 (w/v), or 1:50 (w/v). In a preferred embodiment the ratio of bagasse or straw biomass to solvent in the extraction step is at or about 1:20 (w/v).

In an embodiment, the extraction is carried out at a temperature of at least 20°C. The extraction can be carried out at about 20°C, 21°C, 22°C, 23°C, 24°C, 25°C, 26°C, 27°C, 28°C, 29°C, 30°C, 35°C, 40°C, 45°C, 50°C, 55°C, and 60°C. In a preferred embodiment the extraction is carried out at 23°C.

In an embodiment, the extraction is carried out for at least one hour. The extraction can be carried out for 1 hour, 1.5 hours, 2 hours, 2.5 hours, 3.0 hours, 3.5 hours, 4 hours, 4.5 hours, 5 hours, 5.5 hours, 6 hours, 6.5 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours.

After extraction, the solids can be removed by filtration, centrifugation, or any other process that can be used to separate a solid phase from a liquid phase. The extract can then be concentrated by removal of the solvent. In a preferred embodiment, the solvent is removed by a process involving evaporation of the solvent.

The extracts and compositions obtained by the method disclosed herein can be administered in a variety of unit dosage forms depending upon the method of administration. Dosages for typical modulatory compounds are well known to those of skill in the art. Such dosages are typically advisory in nature and are adjusted depending on the therapeutic context, patient or organ tolerance, etc. The amount of agent contained in the extract adequate to accomplish this is defined as a "therapeutically effective dose." The dosage schedule and amounts effective for this use, i.e., the "administration regimen," will depend upon a variety of factors, including the condition of the heart, the pre-existence or not of damage onset, the pharmaceutical formulation and concentration of active agent, and the like. In calculating the administration regimen for an organ, the mode of administration also is taken into consideration. The administration regimen must also take into consideration the pharmacokinetics, i.e., the extract or composition's rate of absorption, bioavailability, metabolism, clearance, and the like. (See, e.g., the latest Remington's; Egleton and Davis 1997 Peptides 18:1431-1439; Langer 1990 Science 249:1527-1533).

The extracts and compositions may be administered by any convenient means and is contemplated to exhibit beneficial activity when administered in an amount which depends on the case. A broad range of concentrations may be applicable. Considering a subject, for example, from about 0.1 mg to about 1 mg of extract may be administered per kilogram of body weight per day. Administration regimes may be adjusted to provide the optimum beneficial response. For example, several divided doses may be administered daily, weekly, monthly, or other suitable time intervals or the dose may be proportionally reduced as indicated by the exigencies of the situation.

In accordance with these methods, the extract or composition defined in accordance with the present invention may be coadministered with one or more other compounds or molecules. By "coadministered" is meant simultaneous administration in the same formulation or in two different formulations via the same or different routes or sequential administration by the same or different routes. For example, the extract composition may be administered together with an agent in order to enhance its effects. By "sequential" administration is meant a time difference of seconds, minutes, hours or days between the administration of the two types of compositions. These compositions may be administered in any order.

The forms suitable for injectable use include sterile aqueous solutions (water soluble) or dispersions and sterile powders for the extemporaneous preparation of sterile injectable solutions or dispersion. In a preferred embodiment the extract or composition is in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, using a coating such as lecithin, by the maintenance of the required particle size in the case of dispersion and by the use of surfactants. The preventions of the action of microorganisms can be brought about by various antibacterial and antifungal antagonists, for example, parabens, chlorobutanol, phenol, sorbic acid, thimerosal and the like. In many cases, it will be preferable to include isotonic antagonists, for example, sugars or sodium chloride. Prolonged absorption of the injectable forms of the extracts or compositions can be brought about by the use in the extracts or compositions of antagonists delaying absorption, for example, aluminium monostearate and gelatin.

Sterile injectable solutions are prepared by incorporating the extracts or compositions in the required amount in the appropriate solvent with various of the other ingredients enumerated above, as required, followed by filtered sterilisation. Generally, dispersions are prepared by incorporating the various sterilised extracts or compositions into a sterile vehicle which contains the basic dispersion medium and the other required ingredients from those enumerated above. In the case of sterile powders of the extracts for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients of the extract are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions should contain at least 1% by weight of active compound. The percentage of the compositions may, of course, be varied and may conveniently be from about 5% to about 80% of the weight of the unit. The amount of active compound in such beneficially useful compositions is such that a suitable dosage will be obtained. Compositions according to the present invention are prepared so that an oral dosage unit form contains from about 0.1 $\mu$g to about 2000 mg of active compound.

The tablets, troches, pills, capsules and the like may also contain the components as listed hereafter: a binder such as gum, acacia, corn starch or gelatin; excipients such as dicalcium phosphate; a disintegrating antagonist such as corn starch, potato starch, alginic acid and the like; a lubricant such as magnesium stearate; and a sweetening antagonist such as sucrose, lactose or saccharin may be added or a flavoring antagonist such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening antagonist, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound(s) may be incorporated into sustained-release preparations and formulations.

In one aspect, a topical composition may comprise an ointment base, C11-C40 alcohols or C11-C40 acids, such as carboxylic acids, and a polymer. The ointment base may be at least about 50% of the topical composition by weight. The polymer may be substantially soluble in the ointment base.

In some embodiments, the topical composition can further comprise an active agent. In other embodiments, the topical composition can comprise a penetration enhancer.

As one skilled in the art will readily appreciate, the specific ointment base to be used is one that will provide for optimum extract delivery, and, preferably, will provide for other desired characteristics as well, e.g., emollience and occluded-ness. As with other carriers or vehicles, an ointment base should ordinarily be inert, stable, non-irritating and non-sensitizing. Generally, the ointment base may be grouped in four classes: oleaginous bases; emulsifiable bases; emulsion bases; and water-soluble bases. See, e.g., Remington: The Science and Practice of Pharmacy, 19th Ed. (Easton, Pa.: Mack Publishing Co., pp. 1301-1306 (1985). Oleaginous ointment bases include, for example, vegetable oils, synthetic oleaginous esters of carboxylic acids and alcohols, fats obtained from animals, semisolid hydrocarbons obtained from petroleum and the like.

Examples of oleaginous ointment bases include white ointment, yellow ointment, cetyl esters wax, paraffins, petrolatum, white petrolatum, white wax, yellow wax, beeswax, and the like and mixtures thereof. Emulsifiable ointment bases, also known as absorbent ointment bases, contain little or no water and include, for example, hydroxystearic sulfate, anhydrous lanolin, hydrophilic petrolatum and the like and mixtures thereof. Emulsion ointment bases are either water-in-oil (W/O) emulsions, or oil-in-water (O/W) emulsions, and can include, for example, cetyl alcohol, lanolin, glyceryl monostearate, stearic acid and the like and mixtures thereof. Useful water-soluble ointment bases can be those prepared from glycol ethers such as, for example, polyethylene glycols of varying molecular weight, polysorbates and the like and mixtures thereof. Petrolatum may be at a concentration of greater than or equal to 50%, preferably greater than or equal to 70%, for example.

In some embodiments, the C11-C40 alcohol or the C11-C40 carboxylic acid, or combinations thereof may be present in an amount from about 0.1% to about 20%, preferably from 0.1% to 15%, more preferably from 0.1% to 10%, for example, of the composition by weight. In certain embodiments, the C11-C40 alcohol may be a terminally functionalized alkyl alcohol having from 11 to 40 carbon atoms, preferably having from 16 to 40 carbon atoms, more preferably from 20 to 40 carbon atoms, for example.

The general formula of the terminally functionalized alkyl alcohols are: Rn(OH), wherein n is equal to or greater than 11 and equal to or less than 40; wherein R is a saturated or unsaturated hydrocarbon chain. Examples of saturated and unsaturated alkyl alcohols suitable for preparing the compositions of the present embodiment are 1-dodecanol, 1-tridecanol, 1-tetradecanol, 1-pentadecanol, 1-hexadecanol, 1-heptadecanol, 1-octadecanol, 1-nonadecanol, 1-eicosanol, 1-heneicosanol, 1-docosanol, 1-tetracosanol, 1-hexacosanol, 1-heptacosanol, 1-octacosanol, 1-nonacosanol, 1-triacontanol, 1-dotriacontanol, 1-tetratriacontanol, tridecyl alcohol, palmitoleyl alcohol, erucyl alcohol, and combinations thereof. Lanolin alcohol is also suitable.

In certain embodiments, the C11-C40 acids may be terminally functionalized alkyl carboxylic acids having a general formula: Rn(COOH), wherein n is equal to or greater than 11 and equal to or less than 40. In one embodiment, R may be a saturated or unsaturated hydrocarbon chain.

Examples of saturated carboxylic acids suitable for preparing the extracts and compositions of the present embodiment are lauric acid, tridecylic acid, myristic acid, pentadecyclic acid, palmitic acid, margaric acid, stearic acid, nonadecyclic acid, arachidic acid, heneicosylic acid, benenic acid, tricosylic acid, lignoceric acid, pentacosylic acid, cerotic acid, heptacosylic acid, montanic acid, nonacosylic acid, melissic acid, henatriacontylic acid, lacceroic acid, psyllic acid, geddic acid, ceroplastic acid, hexatriacontylic acid, heptatriacontanoic acid, octatriacontanoic acid, and combinations thereof.

Examples of unsaturated carboxylic acids suitable for preparing the compositions of the present embodiment are myristoleic acid, palmitoleic acid, α-linolenic acid, linoleic acid, stearidonic acid, γ-linolenic acid, vaccenic acid, oleic acid, elaidic acid, sapienic acid, eicosapentaenoic acid, dihomo-γ-linolenic acid, arachidonic acid, paullinic acid, gondoic acid, erucic acid, docosahexaenoic acid, docosatetraenoic acid, linoelaidic acid, eicosapentaenoic acid, nervonic acid, sardine acid, herring acid, mead acid, and combinations thereof.

Salts of the unsaturated and saturated carboxylic acids are also suitable for preparing the compositions of the present embodiment. Examples include sodium stearate, magnesium stearate, sodium behenate, sodium oleate, calcium oleate, magnesium oleate, sodium linoleate, and combinations thereof.

The polymer in the compositions may be at a concentration from about 0.1% to about 20%, preferably from about 0.1 to about 15%, more preferably from about 0.1% to about 10%.

The polymer might be poly α-olefins, polyaromatics, or fluoropolymers. Examples of poly α-olefins include polyethylene, polypropylene, polybutylene, poly-1-hexadecene, and poly-1-eicosene. Examples of polyaromatics include polystyrene, substituted polystyrene. Examples of fluoropolymers include polyvinylidene fluoride, polyvinyl fluoride.

The polymer might also be a copolymer. Examples of monomers in the copolymer include vinyl monomers, styrene and functionalized styrene monomers, and α-olefins. In one embodiment, the olefin may comprise α-olefins having at least 11 carbon atoms. It is believed that a copolymer comprising different monomer units is generally preferred for its ability to loosen and disrupt dense and orderly packing of hydrocarbons in petrolatum than a polymer comprising only same monomer units. Furthermore, a copolymer with different monomer units might help dispersion and solubilization of the active agents. Therefore, a copolymer is preferred.

Examples of vinyl monomers include vinyl acetate, maleic acid, and vinyl pyrrolidone. A copolymer of vinyl monomers and α-olefins is preferred. According to the present embodiment, the copolymer needs to be solubilized in the composition base comprising petrolatum. Hydrophobicity of α-olefins increases as the chain length increases. Due to highly hydrophobic nature of the petrolatum base, long chain α-olefins are believed to make the copolymer more soluble in the composition comprising petrolatum. Therefore, copolymers of vinyl monomers and long chain α-olefins are preferred. The long chain α-olefins may have at least 11 carbon atoms, preferably at least 16 carbon atoms, more preferably at least 20 carbon atoms.

Because of the occlusive, emollient, and non-irritating properties of petrolatum, it is desirable to maintain these desirable properties. Therefore, the release enhancing agents, such as alcohols, acids or polymers may be present in an amount from about 0.1% to about 20%, preferably from about 0.1% to about 15%, more preferably from about 0.1% to about 10%, more preferably from about 0.1% to about 5%.

Examples of the cosmetically active agents include vitamins, such as vitamin B, vitamin C, tocopherols (vitamin E), tocopherol derivatives, tocotrienols, vitamin D, vitamin K and derivatives thereof, and suitable combinations thereof, for example.

Moisturizing compounds may include glycerin, urea, methylurea, ethylurea, allantoin, lactates, sugars, methyl glucose ethers, sodium pyrrolidone carboxylic acid, sodium hyaluronate, panthenol, hyaluronic acid, α- and β-hydroxyl acids, such as glycolic acid, lactic acid, mandelic acid, or salicylic acid, or combinations of the suitable moisturizing compounds, for example.

The compositions containing the extracts may further comprise a penetration enhancer, such as tissue penetration enhancers, to enhance release and delivery of the active compound into tissues, such as, for example, skin, ocular, nasal. For these compositions, they might comprise both release enhancing agents of the present embodiment and tissue penetration enhancers. The tissue penetration enhancers may then enhance tissue absorption and penetration of the released active agent. The tissue penetration enhancers might be either in suspended solid or solubilized form in the compositions of the present embodiment.

Tissue penetration enhancers, such as suitable volatile organic solvents, include aliphatic, cycloaliphatic and/or aromatic-aliphatic alcohols, each of which is monohydric or polyhydric, alcohol/water mixtures, saturated and/or unsaturated fatty alcohols which each contains from about 8 to about 18 carbon atoms, saturated and/or unsaturated fatty acids which each contains from about 8 to about 18 carbon atoms and/or esters thereof and the like and mixtures thereof. Useful alcohols are those having from 1 to about 20 carbon atoms, e.g., ethanol, isopropyl alcohol, 1-butanol, 1-octanol, etc.

The present disclosure relates to the discovery of other activities of the extracts rather than only chemical antioxidant activity, i.e. prevention of fat oxidation. The present disclosure describes the biological antioxidant activity, skin and food enzymes inhibition activity capacity, anti-inflammatory, UV filtering capacity and antimicrobial activities, not only against spoilage microorganisms but also skin pathogenic bacteria such as *P. acnes* of the extract. The description of these bioactivities enables the use of straw and/or bagasse extracts for more applications in food, animal feed and cosmetic products, than only as preservative. It enables the use of these extracts for the development of multifunctional ingredients potentially decreasing the amount of preservative ingredient

The preparation of extracts from sugarcane bagasse or straw used a simple method of extraction that is described in Figure 1. The process consists of an ethanolic extraction optimized for the straw and bagasse. The extracts can be used individually or as a mixture thereof to improve the performance.

Plant derived phenolic compounds can neutralize free radicals from reactive oxygen species (ROS), which is important to avoid cell damage oxidative stress scenarios. Hence, there is a huge interest on their use as anti-ageing ingredients. In addition, they have been shown to prevent or control degradation processes, such oxidation during storage, as well as improve the quality and nutritional value of food (e.g. fats, oils). These plants derived extracts are been looked to replace synthetic antioxidants, such as butylated hydroxyanisole (BHA), butylated hydroxytoluene (BHT) and tertbutylhydroquinone (TBHQ) that are normally used and can have a negative impact in human health. Some of these compounds are prohibited in some countries, for example BHA was banned from EU.

Example 1: Preparation of Extracts from Sugarcane Bagasse and Straw

Sugarcane straw and bagasse (*Saccharum officinarum*) were freshly collected in Brazil and shipped to Oporto under refrigerated conditions (-20°C).

Extracts were prepared from straw and bagasse using a process outlined in Figure 1. Straw and bagasse were spread on shallow trays and dried at a constant temperature of 40±2 °C overnight. Dried straw and bagasse were crushed with a cutting mill (SM 100, Retsh) until a 2-4 mm sieve was achieved. Following milling the straw and bagasse samples were extracted independently with two concentrations of ethanol in water (50% and 80% (v/v)) at a ratio of straw or bagasse biomass to solvent of 1:20 (w:v). Extraction was performed at 23°C for 24-48h hours with constant agitation.

The extract was recovered by gauze filtration followed by centrifugation, at 4000 $\times$ g for 10 min at 4 °C. Extracts solution can optionally be concentrated by reverse osmosis. Extracts sugars can be taken from solution using diafiltration technique or by adsorption processes. The total alcohol extract can be concentrated, and the ethanol evaporated under vacuum (150 mbar, 50 °C) through a rota-evaporator. Extracts were used in liquid form or dried by freeze drying and stored at room temperature in the dark until analysis.

Phenolic compounds were identified by LC-ESI-UHR-QqTOF-MS and examination is achieved by using UltiMate 300 Dionex UHPLC, coupled to an Ultra-High Resolution Qq-Time-Of-Flight (UHR-QqTOF) mass spectrometer with 50, 000 Full-Sensitivity Resolution (FSR). Identification of the bioactive compounds was performed using an Acclaim RSLC 120 C18 column (100mm x 2.1 mm, 2.2 μm). The injection volume was 5 μL. Mobile phases were 0.1% aqueous formic acid (solvent A) and acetonitrile with 0.1% formic acid (solvent B). Separation was carried out over 24.5 min under the following gradient conditions: 0 min, 5% B; 22 min, 31.6% B; 23 min, 100% B; 24.6 min, 100% at a flow rate of 0.25 mL/min. Parameters for MS analysis were set using negative ionization mode with spectra acquired over a range from m/z 20 to 1000. The parameters were as follows: capillary voltage, 4.5 kV; drying gas temperature, 200 °C; drying gas flow, 8.0 L/min; nebulizing gas pressure, 2 bar; collision RF, 300 Vpp; transfer time, 120 μs; and prepulse storage, 4 μs. Post-acquisition internal mass calibration used sodium formate clusters with the sodium formate delivered by a syringe pump at the start of each chromatographic analysis. High-resolution mass spectrometry was used to identify the compounds. The elemental composition for the compound was confirmed according to accurate mass and isotope rate calculations designated mSigma. The accurate mass measured was within 5 mDa of the assigned elemental composition, and mSigma values of <20 provided confirmation. Compounds were identified based on its accurate mass [M-H]-. The composition of the extracts obtained from four different batches of straw and bagasse is shown in Table 1. As seen in the table, the extracts display a high diversity of compounds.

TABLE 1: Composition of Bagasse and Straw extracts

| Component | Straw extract | Bagasse extract |
|---|---|---|
| **Total monosaccharides (g/100g dw)** | 5-24.5 | 8-37.3 |
| Cellobiose | 0-2 | 2-8 |
| Glucose | 1-8 | 2-10 |
| Xylose | 0-1 | 0-2 |
| Galactose | 0 | 0-1 |
| Arabinose | 0-2 | 0.1 |
| Fructose | 0-12 | 0-15 |
| Mannitol | 0-6 | 0-6 |
| **Fibers Lignin (g/100g dw)** | 1-14 | 1-15 |
| **Organic acids (g/100g dw)** | 10-30 | 0-3 |
| Malic Acid | 5-12 | 0-4 |
| Succinic Acid | 3-20 | 0-5 |
| Formic Acid | 0-10 | 0-3 |
| **Total Phenolics (g/100g dw)** | 0-6 | 0-5 |
| Chlorogenic acid | 0-1 | 0-1 |
| *trans*-5-Caffeoylquinic acid | 0-1 | 0-1 |
| *trans*-3-Feruloylquinic acid | 0-1 | 0-1 |
| Feruylquinic acid | 0-1 | 0-1 |
| Feruylglucaric acid | 0-1 | 0-1 |
| *p-Coumaric acid* | 0-1 | 0-1 |
| *trans*-Ferulic acid | 0-1 | 0-1 |
| Diosmetin-6-C-glucoside | 0-1 | 0-1 |
| Tricin-7-O-glucuronidesulfate | 0-1 | 0-1 |
| Pinellic acid | 0-1 | 0-1 |
| **Minerals (g/100g dw)** | 1-10 | 0-5 |
| $P^+$ | 0-2 | 0-2 |
| $Mg^{2+}$ | 0-2 | 0-2 |
| $Ca^+$ | 0-2 | 0-2 |
| Na2+ | 0-2 | 0-2 |
| $K^+$ | 1-7 | 0-5 |

Example 2: Bagasse and Straw Extracts are Antioxidant

The antioxidant activity of the soluble extracts was evaluated by ABTS, DPPH, and hemolysis methods (see Figures 2A and 2B). For the non-soluble extracts, the beta-carotene/linoleic method of measuring antioxidant activity was used.

For ABTS radical cation decolorization assay a solution of 2,2'-Azino-bis (3-ethylbenzothiazoline-6-sulfonic acid) radical cation (ABTS•+) was prepared by mixing a 7 mmol/L solution of ABTS and a 2.45 mM solution of potassium persulfate (K2S2O8) at a ratio of 1:1 (v/v). The solution was left in the dark for 16 hours and then diluted with deionized water in order to obtain an initial optical density (OD) of 0.700±0.020 at 734 nm. The samples were diluted to obtain five different concentrations. In a microplate, 15 μL of each of the five sample dilutions were placed in each of two duplicate wells (two wells for each sample dilution) and combined with 200 μL of ABTS•+ per well, followed by incubation for five minutes at 30 °C in a microplate reader. Following the incubation step, the OD of each well was measure at 734 nm. A calibration curve was prepared using Trolox standard solutions. The inhibition percentage was calculated using the following formula:

$$\% \ Inibition = \frac{OD \ \text{ABTS}^{\bullet+} - OD \ sample}{\text{ABTS}^{\bullet+}} \times 100$$

As shown in Figure 2A both bagasse and straw extract demonstrated antioxidant activity in the ABTS assay with the bagasse extract having increased potency as compared to the extract prepared from sugarcane straw.

DPPH radical cation decolorization assay was performed by preparing a stock 600 μM DPPH• solution from DPPH and methanol followed by further diluting the stock solution with methanol to obtain a solution with an OD of 0.600±0.100 at 515 nm. 25 μL of each extract sample was placed in each well of a microtiter plate in duplicate with the respective dilutions and 175 μL of DPPH• solution. The plate was incubated at room temperature for 30 minutes followed by measuring the OD at 515 nm in a microplate reader. The calibration curve was prepared using Trolox standard solutions. Inhibition percentage was calculated through the following formula:

$$\% \ Inibition = \frac{OD \ \text{DPPH}^{\bullet} - OD \ sample}{\text{DPPH}^{\bullet}} \times 100$$

As shown in Figure 2A, the extracts showed similar antioxidant behavior using the DPPH assay as seen in the ABTS assay, with both having activity but the bagasse extract showing increased potency compared to the sugarcane straw extract.

β-carotene based assay was performed in order to determinate the antioxidant activity of non-soluble extracts. β-carotene was dissolved in chloroform at a concentration of 20 mg/mL. A mixture of β-carotene and linoleic acid was prepared by mixing 50 μL of the 20 mg/mL β-carotene solution, 40 μL of linoleic acid, and 530 μL of tween 40. The chloroform was then removed by evaporation under nitrogen flow, and distilled water was added until the OD of the solution was 0.7 at 470 nm. The extract samples were prepared in ethanol and adjusted to a concentration of 50 g/mL and then diluted in 1:1 ratio. 24 μL of extract sample, controls, and standards were placed in wells of a microplate followed by addition of 276μL of the β-carotene/linoleic acid solution, and the plate was incubated at 45°C for 2 hours. The OD was measured at 470nm at 0, 60, and 120 minutes, in a microplate reader. The calibration curve was prepared using Trolox standard solutions. The degradation rate was calculated according to the following formula, where "a" equal the initial absorbance and "b" corresponds to the absorbance measured after 120 minutes:

$$Degradation \ rate = \frac{\ln(\frac{a}{b})}{120}$$

A hemolysis assay was performed as described by Fernandes, JC, et al. (2010) Carbohydrate Polymers, vol. 79, pages 1101-1106. In brief, blood, collected from healthy consenting donors, was centrifuge at 4000 rpm for 10 minutes and the plasma and buffy coat removed, obtaining red blood cells. The red blood cells were then washed three times with phosphate buffered saline (PBS) and resuspended in order to obtain a 2% (v/v) hematocrit. The oxidant agent used in the assay was 2,20-azobis (2-amidinopropane) hydrochloride (AAPH), prepared at a final concentration of 60mM. Three different concentrations, 1%, 0.1%, and 0.01% (w/v), were tested for each extract, and were incubated with AAPH and separately to evaluate the hemolytic capacity of the extracts. All controls and sample tests were prepared in order to obtain a final volume of 650μL and conducted in duplicate. After being incubated at 37 °C for 3 hours, with shaking, 50 μL of each sample was diluted in 950 μL of PBS and water and centrifuged at 4000 rpm for 6 minutes. The supernatant was transferred to a microplate, and the OD measured at 540 nm in a microplate reader. The hemolysis percentage was calculated according to the following formula:

$$\% \ Hemolysis = \frac{OD \ sample}{OD \ control} \times 100$$

As shown in Figure 2B both extracts inhibited hemolysis. Interestingly, in the hemolysis assay the extract prepared from sugarcane straw appeared to be slightly more potent than the extract prepared from bagasse. Nevertheless, both preformed similarly to the ascorbic acid control.

Example 3: Bagasse and Straw Extracts are Anti-inflammatory

Phenolic compounds have been reported to have significant anti-inflammatory activity by cellular functions such as direct interaction with several receptors, modulation of intracellular signal, transcription of genes and modulation of enzymatic activates. The anti-inflammatory activity of the extracts prepared from sugarcane were investigated by assessing the impact of each extract on the secretion of pro-inflammatory cytokines by peripheral blood mononuclear cells (PBMCs) stimulated with lipopolysaccharide (LPS; endotoxin found in the outer membrane of Gram-negative bacteria), following a similar methodology to that previously described by Berker et al. (2014), Journal Periodontology, vol. 84, pages 1337-1345. Briefly, 30 mL of peripheral venous blood was obtained via venipuncture from four healthy volunteers (each donor formed the basis of a single repeat) into heparinized (10U/mL) tubes. PBMCs were isolated using a gradient system with Histopaque 1119 and Histopaque 1077; the tubes were centrifuged at $500 \times$g for 30 minutes at room temperature. The PBMC-rich layer was collected and washed twice with phosphate buffered saline (PBS, pH 7.4), counted, and suspended in RPMI 1640 (Gibco) with 10% foetal calf serum (FCS), L-glutamine (15 mM) and penicillin-streptomycin (5000 units/mL penicillin; 5 mg/mL streptomycin). PBMCs ($1 \times 10^6$ cells/mL) were cultured for 24 hours at 37°C in a 5% $CO_2$ atmosphere. In all sets of experiments (n = 4), a negative control (with PBS) was used. The inflammatory agent used in the assay was LPS from *Escherichia coli* O111:B4, at 10 $\mu$g/mL. Two different concentrations, 1.0 and 0.1 mg/mL, were tested for each extract, and were incubated with LPS and separately to evaluate a potential pro-inflammatory capacity of the extracts.

The levels of TNF-$\alpha$ and IL-6 production were measured by commercially available Enzyme-Linked Immunosorbent Assay (ELISA) from BioLegend (London, UK). The assays were conducted according to the manufacturer's instructions. All samples and standards were run in duplicate and optical density was determined with a microplate reader at 450nm wavelength. Samples above the standard determination range for optical density readings were assayed again and read at an appropriate dilution in order to ensure the levels were within the linear slope of the standard curve. As shown in Figures 3A-3D, sugarcane straw and bagasse demonstrated in vitro anti-inflammatory activity via modulation of inflammatory cytokines secretion leading to overall reduction of pro-inflammatory response (Figure 3A: TNF-$\alpha$ inhibition and Figure 3B: IL-6 inhibition). This activity may be due to the extract inhibition of LPS induced COX-1/2 and/or 5-LOX enzymes.

The ability of the extracts to inhibit COX-1, COX-2, and 5-LOX were evaluated using commercial enzymatic screening assay kits, as follows:

Cyclooxygenase (COX-1 and COX-2) inhibition assay

The extract was tested in triplicates at 0.156, 0.3125, 0.625, 1.25, and 2.5 mg/mL using a commercial COX inhibitor screening assay kit following the protocol recommended by the manufacturer. The COX inhibitor screening assay directly measures the amount of Prostaglandin2$\alpha$ produced in the cyclooxygenase reaction. SC-560 (5-(4-chlorophenyl)-1-(4-methoxyphenyl)-3-(trifluoromethyl)-1H-pyrazole; 3.14 $\mu$M) and DuP-697 (5-bromo-2-(4-fluorophenyl)-3-(4-(methylsulfonyl)phenyl)-thiophene; 2.86 $\mu$M) were run as the positive controls for inhibition of COX-1 and COX-2, respectively. A volume of 10 $\mu$L each of test extract and vehicle were diluted to 20 $\mu$L with 0.1 M Tris-HCl pH 8.0 and pre-incubated with the enzyme at 37°C for 15 minutes prior to the addition of COX substrate AA. The reaction is initiated by addition of 10 $\mu$L of 10 mM AA and the tube was incubated at 37°C for another 2 minutes. Reaction was terminated by addition of 50 $\mu$L of 1 N HCl and saturated stannous chloride. Assays were performed using 100 units of ovine COX-1 and human recombinant COX-2. An aliquot is removed and the prostanoid produced is quantified spectrophotometrically via enzyme immunoassay.

Lipooxygenase (5-LOX) inhibition assay:

The extract was tested in triplicates at 0.156, 0.3125, 0.625, 1.25, and 2.5 mg/mL using the LOX inhibitor screening assay kit following the protocol recommended by the manufacturer. This assay measures the hydroperoxides generated from incubating a 5-LOX enzyme with its substrate, AA. Quercitin (MW = 302.36) was used as the positive control. A volume of 10 $\mu$L each of test extracts and vehicle were pre-incubated with 90 $\mu$L of 5-LOX enzyme in a 96-well plate. The reaction was initiated by addition of 10 $\mu$L of 1 mM AA and the plate was shaken for 5 minutes. Then, 100 $\mu$L of chromogen from the test kit was added to stop enzymic reaction and for colour development. The plate was placed on a shaker for another five minutes and absorbance at 490 nm was measured using microplate reader.

The extracts showed ability to inhibit these three key pro-inflammatory enzymes: 5-lipoxygenase, cyclooxygenase-1 and cyclooxygenase-2 (see Figure 3C: 5-LOX inhibition and Figure 3D: COX-1/2 inhibition).

Example 4: Bagasse and Straw Extracts Contain Skin Enzyme Inhibitors

The effect of bagasse and straw extracts on the activity of enzymes present in skin (collagenase, elastase and tyrosinase) was evaluated (see Figures 4A, 4B, and 4C). The effect of extracts on collagenase activity was determined using a MMP1 inhibitor screening assay kit (colorimetric) (1b139443). MMP inhibitor, MPP substrate, and MPP1 enzyme were prepared. MMP1 enzyme was placed in a flat-bottom microplate following the addition of blank (assay buffer), control, MPP inhibitor, and test samples (various dilutions of bagasse and straw extracts). The plate was then incubated for 1 hour at 37 °C to allow inhibitor/enzyme interaction. Following the incubation step, the reaction was started by the addition of MMP1 substrate and the OD was measured at 412 nm in a microplate reader, in intervals of 10 to 20 minutes. A range of time points, during which the reaction was linear, were selected to obtain the reaction velocity (v) and the inhibitor activity was calculated using the following formula:

$$Inhibitor \% \ activity \ remaining = \frac{V \ inhibitor}{V \ control} \times 100$$

The effect of the bagasse and straw extracts on elastase activity was determined using a neutrophil elastase inhibitor screening kit (fluorometric) (ab118971). Neutrophil elastase (NE) enzyme was prepared according to instructions and added to a flat-bottom microplate. Test inhibitor, inhibitor control, and blank were added to the wells containing enzyme, and the plate was incubated for 5 minutes at 37°C. Following this incubation step, the substrate reaction mix was prepared and added to each well. The plate was incubated in a microplate reader at 37°C for 30 minutes, and the fluorescence measured at a 400/505nm. Two time points in which the reaction was linear were selected, in order to calculate the relative activity for each test inhibitor according to the following formula, in which RFU is the fluorescence generated by hydrolyzation of substrate:

$$\% \ relative \ activity = \frac{\Delta RFU \ test \ inhobitor}{\Delta RFU \ enzyme \ control} \times 100$$

The effect of the bagasse and straw extracts on tyrosinase activity was determined using a tyrosinase inhibitor screening kit (colorimetric) (ab204715). For each reaction, 20 μL of sample solution, a tyrosinase inhibitor working solution (Kojic acid), and a tyrosinase assay buffer were placed in each well of a flat-bottom microplate. Tyrosinase enzyme solution was prepared, and 50 μL added to each well containing test solutions. The plate was incubated at 25 °C for 10 minutes. Following the incubation step, 30 μL of tyrosinase substrate solution was added to each well, the plate was incubated at 25 °C for 1 hour, and the OD measured every 2 to 3 minutes at 510 nm using a microplate reader. After the readings, two time points were selected in order to calculate the slope for all samples (S), inhibition control (IC), and enzyme control (EC) and the relative inhibition calculated through the following formula:

$$\% \ Relative \ Inhibition = \frac{Slope \ EC - Slope \ S}{Slope \ EC} \times 100$$

In addition to oxidation, the accumulation of free radicals can cause several harmful effects in the skin through activation of skin disease-related enzymes, such as tyrosinase, collagenase, and elastase, which can further contribute to skin aging. Thus, the inhibition of these enzymes is expected to prevent some of the skin damage resulting from free radical generation. As shown in Figures 4A-C, the bagasse and straw extracts result in the same level of enzyme inhibition (Figure 4A tyrosinase inhibition, Figure 4B collagenase inhibition, and Figure 4C elastase inhibition) as seen using the reference control enzyme inhibitors used for each. Collagenase is one of the few proteinases capable of degrading collagen. This collagenase mediated degradation of collagen results in a decrease in the elasticity and strength of the skin. Therefore, the inhibition of collagenase spares collagen and thereby prevents skin ageing. In addition, elastase degrades skin elastin. The elastase mediated loss of elastin results in the plasticity and suppleness of skin. Therefore, the inhibition of elastase can prevent the appearance of ageing signs in skin. Tyrosinase is a copper-containing enzyme that catalyzes the first two stages of melanogenesis and can provoke the over-accumulation of melanin in specific parts of the skin. This abnormal tyrosinase activity results in undesirable hyperpigmentation of the skin. Accordingly, inhibition of tyrosinase activity could prevent the formation of skin hyperpigmentation. Further, tyrosinase inhibition is important for the food industry because it prevents the browning of raw fruits, vegetables, and beverages.

Example 5: Bagasse and Straw Extracts have Antimicrobial Activity

The antimicrobial activity of the extracts was evaluated by measuring the minimum inhibitory concentration (MIC) and the minimum bactericidal concentration (MBC) of the extracts using the methods of the clinical and laboratory standards institute for aerobic (M07-A9) and anaerobic bacteria (M11-A8). From a previously filtered 3% stock solution a series of extract dilutions were prepared (2%, 1.5%, 1%, 0.75%, 0.5%, 0.25%, 0.125%, and 0.0625% (w/v)). A commonly used preservative, 2-phenoxyethanol was tested under the same conditions as a positive control.

MHB growth media was used for aerobic microorganisms and Wilkins Chalgren Broth (WCB) was used for anaerobic microorganisms. The microorganisms were inoculated at 2% (v/v) from an inoculum with 0.5 McFarland standard (ca. $10^8$ CFU/mL) and then adjusted in order to obtain a final inoculum of $10^5$ CFU/mL in the testing solutions. Controls were performed with the culture media, extract mother solutions, and culture media inoculated with each microorganism without extracts. Incubations were performed in a 96-well microplate for 24 hours at 37 °C, except for *P. acnes* incubations which were performed for 48 h with a parafilm cover over the microplates to provide anaerobic conditions. The OD of each well (experimental and control) was measured each hour at 660 nm. All measurements were performed in triplicate. Afterwards, the concentrations for which no growth was detected were plated in MHA and Wilkins Chalgren Agar (WCA) accordingly, in order to verify the MBC. MBC and MIC were determined by the analysis of maximum absorbance registered, delay of the exponential phase entry, and the calculation of the specific growth rate. The specific growth rate was obtained by the determination of the slope of the trend line of the OD660 over log phase of the growth curves. As shown in Table 2, the bagasse and straw extracts were shown to have promising antimicrobial activities against reference microorganisms strains responsible for contamination and spoilage of food and cosmetic products.

Table 2. MBC and MIC from bagasse and straw extracts (50% ethanol) and 2-phenoxyethanol of contaminant microorganisms.

| Microorganism | Bagasse | | Straw | | Phenoxyethanol |
|---|---|---|---|---|---|
| | MBC | MIC | MBC | MIC | MBC |
| *E. coli* | ND | 0.25% | ND | 0.25% | 3% |
| *P. aeruginosa* | ND | ND | ND | 2% | 0.3% |
| *MSSA* | 3% | 0.25% | ND | 0.25% | 0.3% |
| *C. albicans* | ND | 3% | ND | 3% | 3% |
| ND - inhibition not detected; MBC: Minimal Bactericidal Concentration; MIC: Minimal Inhibitory Concentration | | | | | |

A preservative test challenge following the European Pharmacopeia Protocol 7.0 for topical products was executed to evaluate the potential of the bagasse and/or straw extracts to be used in the formulation of a preservative ingredient for cosmetic products. The efficacy of antimicrobial preservation was determined using the protocol of the European Pharmacopeia 7.0. Extracts were tested at 1%, 2% and 3% (w/v) preliminary tested in a micellar water.

Straw and bagasse extracts obeyed the criteria of the protocol in different ways - straw was the most effective and a mixture of both extracts were found to have a higher inhibition effect than the individual extracts (see Figures 5A-D).

In addition, these extracts were tested against P. acnes the bacteria responsible for the development of the skin infectious disease acne, reading optical densities and MBC of 3% and MIC of 0.0625% were found for straw extracts.

Preservative effect determination

The present disclosure shows that the straw extract also has biological antioxidant activity at 0.1% (m/v), a level that is comparable with ascorbic acid (Figure 2). This is besides having ABTS and DPPH chemical antioxidant activity.

UV adsorbing extracts

The present disclosure shows that sugarcane straw and bagasse extracts have UV radiation absorption capacity. UV radiation is considered the most frequent irritant factor for human skin cells and the most external stress inducing factors in extrinsic skin ageing. Oxidative stress is generated during exposure to UV. Even though the human skin is able to survives the daily oxidative hits of 105, ROS still causes oxidative damage in cellular components, such cell walls, lipid membranes, mitochondria and DNA, provoking injury in the connective tissue of the skin. As already mentioned, the aromatic structure of polyphenols is the significant feature in oxidative stress, namely in preventing the formation and scavenging of ROS and reactive nitrogen species (RNS) and minimize the effects of UV stress and harmful compounds in skin ageing. Hence, sugarcane straw and bagasse extracts have potential application for the development of anti-ageing cosmetic products.

In an embodiment the extract may be present at concentrations greater than 1 mg/mL to have antioxidant activity, greater than 1 mg/mL to have anti-inflammatory activity and greater than 3mg/mL to inhibit the activity of skin enzymes such as elastase, tyrosinase and collagenase.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof.

The above described embodiments are combinable.

**Claims**

1. A method of preparing an extract from sugarcane bagasse or sugarcane straw comprising:

   obtaining a dried sugarcane bagasse or a dried sugarcane straw;
   milling the dried bagasse or the dried straw;
   mixing the milled bagasse or straw with a solution comprising a solvent to form a mixture;
   stirring the mixture;
   separating liquid fraction and solid fraction of the mixture;
   concentrating the liquid fraction to obtain an extract;
   optionally drying the liquid fraction to obtain a dry extract.

2. The method according to the preceding claim, wherein the bagasse or the straw are dried at a temperature not less than 30 °C, preferably at a temperature of about 40 °C.

3. The method according to any of the preceding claims, wherein the dried bagasse or the dried straw is milled to a size ranging from 2 mm to 4 mm.

4. The method according to any of the preceding claims, wherein the solvent is selected from acetone, dichloromethane, ethanol, methanol, or combinations thereof.

5. The method according to any of the preceding claims, wherein the solution comprising solvent comprises ethanol.

6. The method according to any of the preceding claims, wherein the mixture is stirred for at least 24 hours.

7. The method according to any of the preceding claims, wherein the mixture is stirred at a temperature ranging from 20°C to 22 °C.

8. The method according to any of the preceding claims, wherein the liquid fraction and the solid fractions are separated by filtration, preferably filtration through gauze.

9. An extract obtained by the method according to any of the preceding claims.

10. The extract according to the preceding claim for use in medicine or veterinary.

11. The extract according to any of the claims 9-10 for use in the treatment or prevention of inflammatory diseases or inflammatory symptoms, in particular in skin inflammatory diseases.

12. The extract according to any of the claims 9-11 for use in the inhibition or reduction of skin enzymes activity, as anti-ageing agent, the skin protection against UV light damage, as antioxidant, as an anti-microbial agent and/or as an anti-acne agent.

13. The extract according to any of the claims 9-12 comprising from about 2% (wt/wt) to 8% (wt/wt) cellobiose, from about 2% (wt/wt) to 10% glucose(wt/wt), not more than about 2%(wt/wt) xylose, not more than 1% (wt/wt) galactose, not more than 0.1%(wt/wt) arabinose, not more than 15% (wt/wt) fructose, and not more than 6%(wt/wt) mannitol.

**14.** The extract according to any of the claims 9-13 further comprising not more than 4% (wt/wt) malic acid, not more than 5% (wt/wt) succinic acid, and not more than 3% (wt/wt) formic acid.

**15.** The extract according to any of the claims 9-14 further comprising not more than 1% (wt/wt) chlorogenic acid, not more than 1% (wt/wt) trans-5-caffeoylquinic acid, not more than 10% (wt/wt) trans-3-feruloylquinic acid, not more than 1% (wt/wt) feruylquinic acid, not more than 1% (wt/wt) feruylglucaric acid, not more than 1% (wt/wt) p-coumaric acid, not more than 1% (wt/wt) rans-ferulic acid, not more than 1% (wt/wt) diosmetin-6-C-glucoside, not more than 1% (wt/wt) tricin-7-O-glucuronidesulfate, and not more than 1% (wt/wt) pinellic acid.

**16.** The extract according to any of the claims 9-15 further comprising not more than 2% (wt/wt) cellobiose, from about 1%(wt/wt) to 8% (wt/wt) glucose, not more than about 1% (wt/wt) xylose, not more than 2% (wt/wt) arabinose, not more than 12% (wt/wt) fructose, and not more than 6% (wt/wt) mannitol.

**17.** The extract according to any of the claims 9-16 further comprising about 5 % (wt/wt) to 12% (wt/wt) malic acid, from about 3% (wt/wt) to about 20% (wt/wt) succinic acid, and not more than 10% (wt/wt) formic acid.

**18.** The extract according to any of the claims 9-17 further comprising not more than 1% (wt/wt) chlorogenic acid, not more than 1% (wt/wt) trans-5-caffeoylquinic acid, not more than 1% (wt/wt) trans-3-feruloylquinic acid, not more than 1% (wt/wt) feruylquinic acid, not more than 1% (wt/wt) feruylglucaric acid, not more than 1% (wt/wt) p-coumaric acid, not more than 1% (wt/wt) trans-ferulic acid, not more than 1% (wt/wt) diosmetin-6-C-glucoside, not more than 1% (wt/wt) tricin-7-O-glucuronidesulfate, and not more than 1% (wt/wt) pinellic acid.

**19.** A composition comprising an active amount of the extract according to the previous claims 9-18 and suitable excipients, in particular a pharmaceutical composition or a cosmetic composition.

**20.** The composition according to the preceding claim, wherein the concentration of the extract is at least 1.25 mg/mL.

**21.** The composition according to any of preceding claims 19-20 for topical administration, preferably the topical composition comprises a penetration enhancer.

**22.** The composition according to any of preceding claims 19-21 further comprising an active agent.

**23.** Use of the extract described in the claims 9-16 as a preservative, in particular as a cosmetic preservative or food preservative.

**24.** A foodstuff or a food composition comprising the extract according to the previous claims 9-16.

```
┌─────────────────────────────┐
│ Straw and bagasse drying and │
│           milling            │
└─────────────────────────────┘
               ▼
┌─────────────────────────────┐
│  Extraction step with solvents │
└─────────────────────────────┘
               ▼
┌─────────────────────────────┐
│  Separation of liquid and solid │
│            fractions          │
└─────────────────────────────┘
               ▼
┌─────────────────────────────┐
│  Concentration steps and sugar │
│        removal and            │
└─────────────────────────────┘
               ▼                        ┌─────────────────────────────┐
┌─────────────────────────────┐         │      Solvent recovery        │
│     Optional drying step     │         └─────────────────────────────┘
└─────────────────────────────┘
```

**Fig. 1**

**Fig. 2A**

**Fig. 2B**

## TNF-α

**Fig. 3A**

# IL-6

**Fig. 3B**

# 5-LOX inhibition

## Bagasse

## Straw

**Fig. 3C**

# COX-1/2 inhibition

| Enzyme | Bagasse | Straw | Reference inhibitor |
|--------|---------|-------|---------------------|
| COX-1 | 74.31% | 88.97% | 75.89%** |
| COX-2 | 83.70% | 91.44% | 87.05%*** |

*2.5 mg/mL; **SC-560 - 3.14 µM; ***Dup-697 - 2.86 µM

**Fig. 3D**

**Fig. 4A**

**Fig. 4B**

**Fig. 4C**

**Fig. 5A**

# Antimicrobial activity – preservative potential

## Candida albicans ATCC 10231

Legend:
- Bagasse + Straw (2%)
- Straw 2%
- Bagasse 2%

X-axis: Log (UFC/mL) Reduction
Y-axis: Time (days)

*Preservative challenge test (EUROPEAN PHARMACOPOEIA 7.0 5.1.3. Efficacy of antimicrobial preservation)*

**Fig. 5B**

# Antimicrobial activity – preservative potential

## Aspergillus brasiliensis ATCC 16404

Legend:
- Bagasse + Straw (2%)
- Straw 2%
- Bagasse 2%

X-axis: Log (UFC/mL) Reduction
Y-axis: Time (days)

*Preservative challenge test (EUROPEAN PHARMACOPOEIA 7.0 5.1.3. Efficacy of antimicrobial preservation)*

**Fig. 5C**

Europäisches
Patentamt

European
Patent Office

Office européen
des brevets

## EUROPEAN SEARCH REPORT

Application Number

EP 19 21 2747

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/028506 A1 (PROD MAKERS AUSTRALIA PTY CO LTD) 14 February 2019 (2019-02-14) * claims 1-3, 6-9, 12-16, 18, 27 * * page 54, line 26 - page 55, line 3 * ----- | 1-22,24 | INV. A23L3/3472 A61K8/9794 A61K36/899 A61P17/10 |
| X | WO 2014/032100 A1 (PHYTOLIN PTY LTD [AU]) 6 March 2014 (2014-03-06) * claims 3,9-15,17-19 * * paragraphs [0001], [0010], [0012], [0019], [0023], [0044], [0074] * ----- | 1-22,24 | A61P17/18 A61Q17/04 A61Q19/08 A23L29/206 A23L33/105 |
| X | EP 2 209 392 A1 (HORIZON SCIENCE PTY LTD [AU]) 28 July 2010 (2010-07-28) * claims 1-11 * * paragraphs [0037] - [0057] * ----- | 1-24 | |
| A | PAYET B ET AL: "Comparison of the Concentrations of Phenolic Constituents in Cane Sugar Manufacturing Products with Their Antioxidant Activities", JOURNAL OF AGRICULTURAL AND FOOD CHEMISTRY, AMERICAN CHEMICAL SOCIETY, BOOKS AND JOURNALS DIVISION¦, US, vol. 54, no. 19, 1 January 2006 (2006-01-01), pages 7270-7276, XP008133517, ISSN: 0021-8561, DOI: 10.1021/JF0608080 [retrieved on 2006-08-29] * the whole document * ----- | 1-24 | |

TECHNICAL FIELDS SEARCHED (IPC)

A23L
A61P
A61Q
A61K

The present search report has been drawn up for all claims

| Place of search | Date of completion of the search | Examiner |
|---|---|---|
| The Hague | 12 February 2021 | Heirbaut, Marc |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 2747

This annex lists the patent family members relating to the patent documents cited in  the above-mentioned European search report.
The members are as contained in the European Patent Office EDP file on
The European Patent Office is in no way liable for these particulars which are merely given for the purpose of information.

12-02-2021

| Patent document cited in search report | | | Publication date | Patent family member(s) | | | Publication date |
|---|---|---|---|---|---|---|---|
| WO 2019028506 | A1 | | 14-02-2019 | AU | 2018315055 | A1 | 19-03-2020 |
| | | | | CN | 111194219 | A | 22-05-2020 |
| | | | | EP | 3664827 | A1 | 17-06-2020 |
| | | | | SG | 11202000824U | A | 27-02-2020 |
| | | | | US | 2020215145 | A1 | 09-07-2020 |
| | | | | WO | 2019028506 | A1 | 14-02-2019 |
| WO 2014032100 | A1 | | 06-03-2014 | AU | 2013308395 | A1 | 05-03-2015 |
| | | | | AU | 2017200670 | A1 | 23-02-2017 |
| | | | | BR | 112015004520 | A2 | 04-07-2017 |
| | | | | CN | 104780987 | A | 15-07-2015 |
| | | | | EA | 201590458 | A1 | 30-10-2015 |
| | | | | EP | 2890467 | A1 | 08-07-2015 |
| | | | | EP | 3569298 | A1 | 20-11-2019 |
| | | | | JP | 6239622 | B2 | 29-11-2017 |
| | | | | JP | 2015528448 | A | 28-09-2015 |
| | | | | PH | 12015500408 | A1 | 20-04-2015 |
| | | | | PH | 12018502169 | A1 | 24-06-2019 |
| | | | | SG | 11201501044R | A | 28-05-2015 |
| | | | | US | 2015201660 | A1 | 23-07-2015 |
| | | | | US | 2019183149 | A1 | 20-06-2019 |
| | | | | WO | 2014032100 | A1 | 06-03-2014 |
| EP 2209392 | A1 | | 28-07-2010 | AU | 2008307137 | A1 | 09-04-2009 |
| | | | | BR | PI0817862 | A2 | 07-10-2014 |
| | | | | CA | 2700850 | A1 | 09-04-2009 |
| | | | | CN | 101815444 | A | 25-08-2010 |
| | | | | EP | 2209392 | A1 | 28-07-2010 |
| | | | | ES | 2454649 | T3 | 11-04-2014 |
| | | | | JP | 2010540566 | A | 24-12-2010 |
| | | | | RU | 2010117226 | A | 10-11-2011 |
| | | | | US | 2010291006 | A1 | 18-11-2010 |
| | | | | US | 2012141390 | A1 | 07-06-2012 |
| | | | | WO | 2009043097 | A1 | 09-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EGLETON ; DAVIS.** Remington's. 1997, 1431-1439
- **LANGER.** *Science,* 1990, vol. 249, 1527-1533
- Remington: The Science and Practice of Pharmacy. Mack Publishing Co, 1985, 1301-1306
- **FERNANDES, JC et al.** *Carbohydrate Polymers,* 2010, vol. 79, 1101-1106
- **BERKER et al.** *Journal Periodontology,* 2014, vol. 84, 1337-1345